**Europäisches Patentamt**

**European Patent Office**

**Office européen des brevets**

(19)

(11) Publication number: **0 070 652**
**B1**

(12)

# EUROPEAN PATENT SPECIFICATION

(45) Date of publication of patent specification: **14.01.87**

(51) Int. Cl.⁴: **A 61 B 1/06, G 02 B 23/08**

(21) Application number: **82303595.1**

(22) Date of filing: **09.07.82**

(54) Hard endoscopes.

(30) Priority: **16.07.81 JP 111448/81**

(43) Date of publication of application:
**26.01.83 Bulletin 83/04**

(45) Publication of the grant of the patent:
**14.01.87 Bulletin 87/03**

(84) Designated Contracting States:
**AT BE CH DE FR GB IT LI NL SE**

(56) References cited:
**US-A-3 297 022**
**US-A-4 072 147**
**US-A-4 319 563**

(73) Proprietor: **OLYMPUS OPTICAL CO., LTD.**
**43-2, 2-chome, Hatagaya Shibuya-ku**
**Tokyo 151 (JP)**

(72) Inventor: **Hashiguchi, Toshihiko**
**No. 3-15, Seishin 8-chome**
**Sagamihara-shi Kanagawa-ken (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

## Description

This invention relates to rigid endoscopes, i.e. endoscopes having a rigid outer housing, of the type in which projecting ends of light guides having light distribution characteristics project illumination widely dispersed from a tip of a part inserted into a body cavity.

Generally, in an endoscope the part inserted into a body cavity to make an observation, attempt a diagnosis, or effect treatment therapy by using a forceps, both an observing optical system and an illuminating optical system are provided at the tip of the inserted part, so that illumination may be projected toward the object of interest, from the end of this illuminating optical system, and so enable reflected light to be studied via the observation system.

Light guides in the form of glass-fibre bundles are used for the above-mentioned illumination optical system. However, if the illuminating range projected from the ends of the light guides is narrow, the region which can be observed via the observing optical system will be limited, and therefore there may be a part in which the object position cannot be well observed, and diagnosis will be impeded.

One object of the present invention is to provide a rigid endoscope wherein illumination projected from light-guide fibres are widely dispersed and the light distribution characteristics are favourable.

In accordance with the present invention, there is provided an endoscope having a rigid insertion sheath housing an eccentrically-positioned inner tube containing an observing optical system, a bunch of light-guide fibres for transmitting illuminating radiation to be projected from their end surfaces partially surrounding the outer periphery of the inner tube, characterised in that the inner tube has a partially-curved tip surface made by cutting a first member with an outer part-spherical end face to form an inclined viewing aperture and the outer sheath also has a partially-curved tip surface made by cutting a second member with an inner part-spherical surface to leave an inclined end face aperture that is substantially filled by said inner tube and said optical fibres.

The invention will now be described with reference to the drawings, in which:—

Figure 1 is a perspective view of the tip of the insertion sheath of a conventional known endoscope;

Figure 2 is a sectional view of the known tip shown in Figure 1;

Figure 3 is a side view of one exemplary embodiment of an endoscope constructed in accordance with the present invention;

Figure 4 is a magnified sectional view showing the tip of the embodiment illustrated in Figure 3;

Figures 5(a), 5(b) and 5(c) are respective sectional views on the lines A—A, B—B and C—C of Figure 4;

Figure 6 is a sectional view showing a section of an unshaped sheath tip blank before a cutting operation in a preferred method of manufacture;

Figure 7 is a sectional view showing the finished sheath tip member;

Figure 8 is a sectional view showing a section of an unshaped inner tube blank for use inside the insertion sheath of Figure 6, before a cutting operation in a preferred method of manufacture;

Figures 9(a), 9(b) and 9(c) are respectively an end view, sectional side view and perspective view respectively of the formed inner tube;

Figures 10(a), 10(b) and 10(c) are respectively an exploded end view, an exploded side view and assembled perspective view showing a filler member fitted on the inner tube;

Figure 11 is a sectional side view showing a preferred method of inserting light guide fibres and the inner tube into the outer sheath;

Figure 12 is a sectional side view showing a step in the forming of light guide fibre end surfaces;

Figure 13 is a sectional side view showing a preferred method of attaching a cover glass to the inner tube;

Figures 14(a), 14(b) and 14(c) are respectively explanatory views showing in pairs the shapes of light guide fibres in the respective arranged positions in the first embodiment as seen in plan and side views;

Figure 15 is an explanatory side view showing directions of illuminating light-paths;

Figure 16 is an explanatory plan view showing directions of the illuminating light paths;

Figure 17 is a sectional side view showing the structure of a second exemplary embodiment of the present invention; and

Figure 18 is a sectional side view showing the structure of a further exemplary embodiment of the present invention.

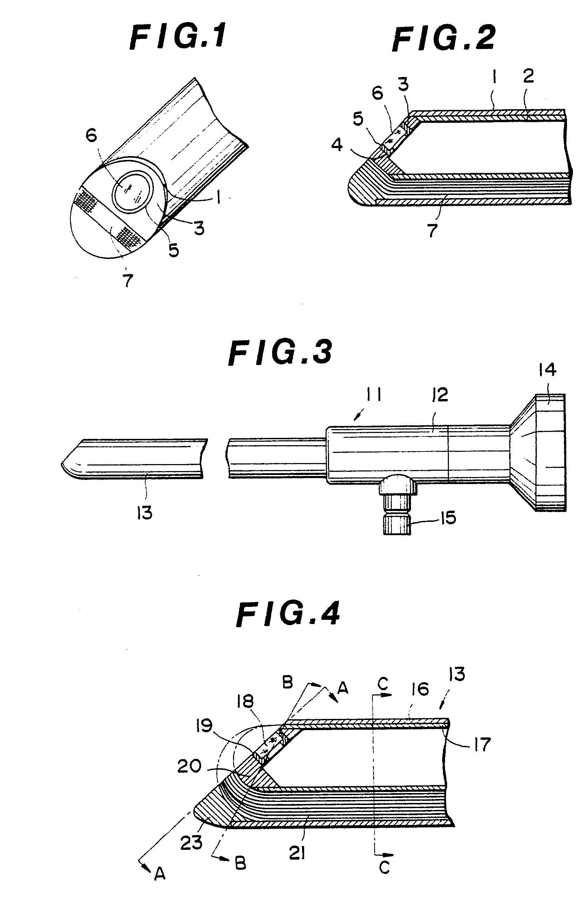

Prior to explaining the embodiments of the present invention, a conventional known example of a hard endoscope will be described with reference to Figures 1 and 2, which show a perspective view and section of a known rigid endoscope having an inner tube 2 incorporating an observing optical system (not illustrated) eccentrically positioned within an outer sheath probe part, an inner tube tip member 3 forming an inclined surface at right angles to the longitudinal axis in a transverse direction with an observing optical system arranged at the tip to give a perspective visual field direction. An opening 4 is made in this inner tube tip member 3, and is fitted with a cover glass 6 in a frame 5. Further, the above mentioned inner tube tip member 3 is formed to be of an arcuate surface on the outer periphery on the sides of light guide fibres 7 inserted and arranged on the outer periphery of the inner tube 2 so as to curve and arrange the projecting end faces of the light guide fibres 7 in the perspective visual field direction.

Thus, in the structure of the tip of the inserted part of this known perspective type rigid endoscope, the area through which the illuminating light paths are projected from the ends of the light guide fibres 7 is small, the illuminating light paths are not well diffused from the projecting end faces, the illuminated field is narrow and the light distribution characteristics are low.

One object of the present invention is to provide a construction which avoids the described problems of this known example.

Embodiments of the present invention will now be described with reference to Figures 3 to 18.

Figures 3 to 5 show a first embodiment of the present invention. This embodiment relates to a perspective type rigid endoscope 11 comprising an operating part 12 and an insertion probe sheath 13. The operating part 12 has an eyepiece part 14 and a light guide cable connecting joint 15. The probe sheath 13 has an inner tube 17 provided eccentrically within an outer housing tube 16. An observing optical system (not illustrated) is contained in this inner tube 17.

An inner tube tip member 20 supporting and fixing a frame 19 holding a cover glass 18 is secured by a bonding agent or by soldering to an opening at the tip of the inner tube 17. This inner tube tip member 20 is hemispherical, with the outside diameter of the inner tube 17 as a diameter, and is partly cut to form a perspective visual field surface. Many light guide fibres 21 transmit and project illuminating light arranged in a substantially meniscus-shaped section formed between this inner tube 17 and the outer tube 16 eccentrically containing the inner tube 17. These light guide fibres 21 are squeezed and converged by a saddle-shaped filler member 22 that is fixed on the outer periphery of the inner tube 17 near the tip and strongly fixed as shaped so as to have various curvatures in the respective parts of the projecting end faces.

The light guide fibres 21 are cut and ground so that the end faces may be flush with the perspective visual field surface, their inner peripheral surfaces are curved near the light-projecting end faces along the outer periphery of the inner tube tip member 20, and their outer peripheral faces are contained so as to contact the inner surface of the outer tube 16 to which a tip member 23 having a surface flush with the perspective visual field surface is attached.

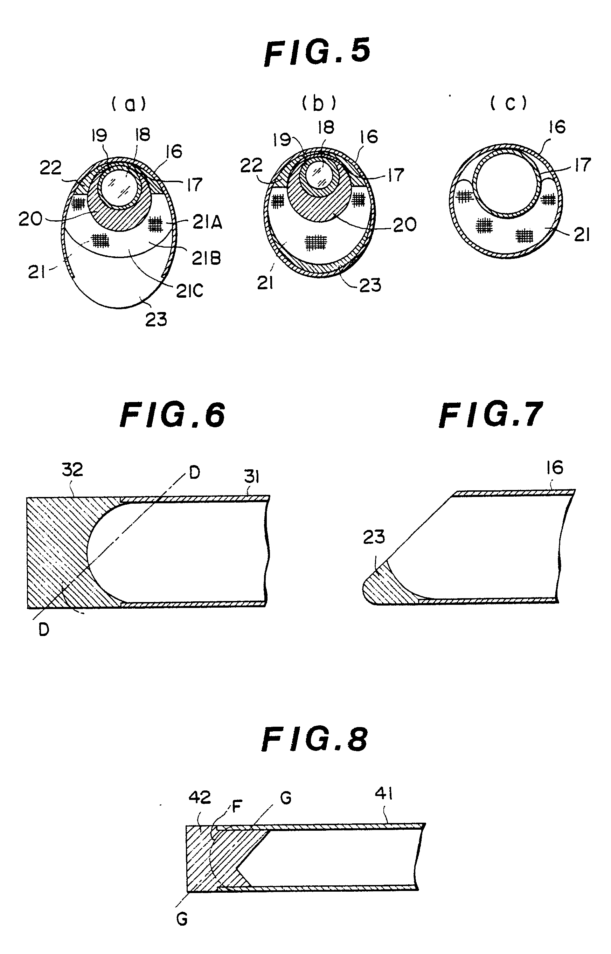

The hemispherical centre of the above mentioned inner tube tip member 20 is eccentric from the hemispherical centre of the tip of the outer tube 16 and the light guide fibres 21 are contained and constrained so as to have various curvatures in the respective parts of the light-projecting end face. These light guide fibres 21 as contained are shown in Figure 5. Figures 5(a), 5(b) and 5(c) are explanatory sectional views respectively showing the section shown on the line A—A of Figure 4 (which shows the perspective visual field surface substantially at the tip of the inserted probe sheath 13), the section on the line B—B of Figure 4, and the section on the line C—C of Figure 4.

The steps of working and assembling the endoscope tip having such structure will be explained with reference to Figures 6 to 13.

Figures 6 and 7 show respectively a preform blank and completed product as made in a preferred method of manufacturing the outer tube 16 of the inserted probe sheath 13.

First, as shown in Figure 6, a columnar tip member 32 having a hemispherical recess formed therein is fixed by soldering, or by a bonding agent, to an open tip of a cylindrical outer tube 31, and is then cut on a line D—D so as to have a predetermined perspective visual field surface, and is rounded-off along the illustrated broken-line curve at the front corner to form a tip member 23 on the residual outer tube 16, as shown in Figure 7.

The preferred method for formation of the inner tube 17 and inner tube tip member 20 will now be explained with reference to Figures 8 to 10.

A cylindrical inner tube 41 having an open tip has a columnar inner tube tip member 42 having a reduced diameter equal to the internal diameter of this inner tube 41, to fit therein, and the remainder of the tip member 42 has a diameter equal to the outer periphery of the inner tube 41. A surface parallel with the predetermined perspective visual field surface on which a cover glass 18 (See Figure 4) is to be fitted and fixed, and a cut surface substantially at right angles with this preformed surface are cut into the rear end of the tip part, to be inserted into the inner tube 41.

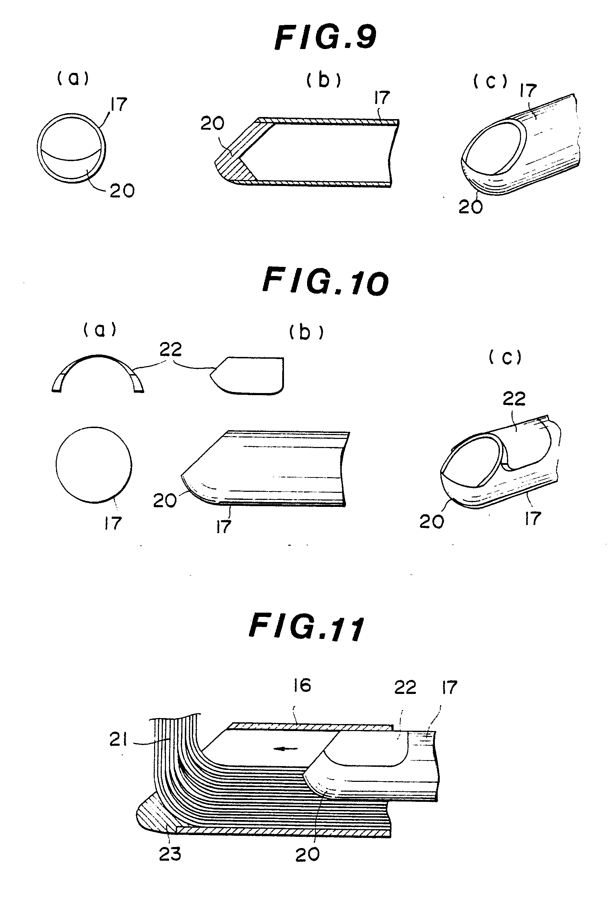

This inner tube tip member 42 and the inner tube 41 are fitted together and fixed, and then hemispherically cut and shaped as shown by the broken-line curve F, before being cut off on the line G—G to define the visual field surface, as is shown in Figures 9(a), 9(b) and 9(c), which respectively show an end view, a sectional side view and a perspective view of the inner tube 17 with the inner tip member 20 attached and fixed.

A filler member 22 of saddle-shape, as shown by the views in Figure 10, is fixed, e.g. by soldering, to the upper part of the inner tube 17 at its tip to lie adjacent to the outer tube 16 when the inner tube is contained eccentrically in the outer tube 16.

The inside and outside surfaces of the filler member are formed so as to respectively have the same curvatures as of the outer periphery of the inner tube 17 and the inner wall of the outer tube 16. As the centres of these curvatures are eccentric from each other, the filler member is thinnest in the middle part and is thickest at each peripheral side part. This filler member 22 is fixed to lie flush with the front end of the visual field surface as shown in Figure 10(c). Both peripheral side parts, thickened as mentioned above, are shaped near the visual field surface so as to have an appropriate curvature required to improve the light distribution characteristics of light from the light-projecting end surfaces of the guide fibres 21, as can be seen in Figures 10(b) and 10(c).

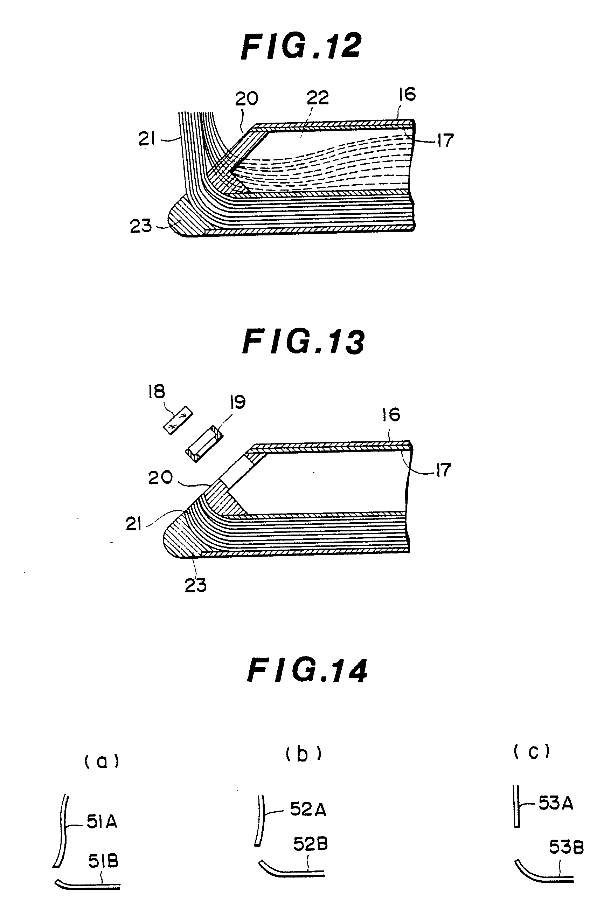

A shaped-bunch of light guide fibres 21 are inserted into the outer tube 16, as shown in Figure 11 and then the inner tube 17 is inserted into the outer tube 16 as indicated by the arrow and fixed in a position in which both visual field surfaces of the outer tube 16 and inner tube 17 are flush with each other, as shown in Figure 12, the individual light guide fibres 21 being fixed at the visual field surface by the pressure from the filler member 22, so that the fibres can be cut and their end faces ground to be optically flat. The pressure on the light guide fibres 21, squeezed near their tips by the filler member 22, are arranged so as to have various curvatures in respective exit zones of the end face.

A hole is made in the inner tube tip member 20, and a cover glass 18, fitted and fixed in a frame 19, is inserted into this hole and fixed with a bonding agent or the like (see Figure 13).

In the first described embodiment, completed by the method steps set out above, the light guide fibres 21 contained in the substantially meniscus-shaped spaced between the outer tube 16 and the inner tube 17 eccentrically fitted therein follow different paths at their ends, as shown in Figure 14.

Figures 14(a), 14(b) and 14(c) show respective shapes near the projecting ends, as seen in plan and from the side, the light guide fibres 21 having the projecting ends in the positions shown by the respective reference numerals 21A, 21B and 21C in Figure 5(a) being shaped on paths as shown by 51A and 51B; 52A and 52B; and 53A and 53B respectively.

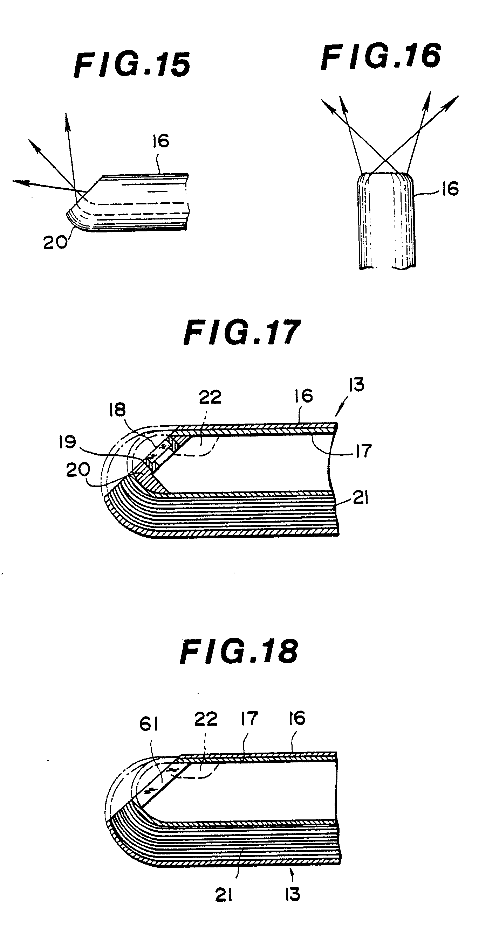

Therefore, in this case, the light distribution characteristics of the illuminating light paths projected from the ends of the light guide fibres 21 will be as shown in Figures 15 and 16.

Figures 15 and 16 show respectively the light distribution characteristics of the bunch of light guide fibres 21 as seen respectively from the side and from below. The light guide fibres 21 are so arranged and contained as to be able to project light uniformly over a wide range, in various directions from respective zones of the projecting surfaces as indicated by the arrows.

Thus, when using a rigid endoscope with a wide light distribution characteristic, as provided by this embodiment, a wide range will be illuminated and all the organs and internal details within the body cavity will be able to be well observed.

Further, using the above described manufacturing methods, the number of steps can be reduced compared to conventional manufacture, and the number of component parts can be also reduced, to the advantage of both manufacture and maintenance.

Figure 17 shows structural details of a second embodiment of the present invention, in which there are used an outer tube member and inner tube member each hemispherical at their tip, as shown by broken-lines, and cut to avoid the need to form a specially shaped tip 23. The outer tube 16 and inner tube 17 are each formed by being partly cut at these tips, and the inner tube residual tip 20 is of substantially the same shape as that of the first described embodiment is inserted and fixed at the tip of the inner tube 17. This inner tube tip member 20 is provided with an internal face parallel with the visual field surface to give a wall of a predetermined thickness, and a further face substantially vertical to this surface, these faces being formed as illustrated by cutting a bar shaped to fit the inner surface of the inner tube 17 and being hemispherical at the tip. A hole is made in the wall parallel with the visual field surface, in which a frame 19 holding a cover glass 18 is fitted and fixed. The saddle-shaped filler member 22 pressing upon the bunch of fibres 21 to improve the light distribution characteristics is fixed to the outer periphery of the inner tube 17 tip in the same manner as described above with reference to the first embodiment.

The tip member 23 of the first embodiment, is made unnecessary and it is relatively easy to form and fix the inner tube tip member 20.

In some cases, the inner tube tip 20 can be made with a cut surface forming the perspective visual field surface, if the material of the tube has the required strength to eliminate the need for internal tip support.

In this variant form of the second embodiment, not only are excellent light distribution characteristics obtained, but also the numbers of component parts and manufacturing steps can be reduced, and therefore the product cost lowered.

In a third exemplary embodiment, a cover glass 61 having a predetermined thickness is shaped to fit directly in the part-spherical inner tube cut at the tip to provide the perspective visual field surface, as shown in Figure 18, there being no inner tube tip member 20.

Thus, the number of component parts and manufacturing steps is still further reduced and the product cost can be made even lower.

In the above described embodiments, the rigid endoscope tip is formed by partly cutting an inner tube 17 and outer tube 18 that each have hemispherically-shaped tips. However, even if the perspective visual field surface is formed by cutting an inner tube 17 and outer tube 18 each having a conical or a parabolic curved surface to form the hard endoscope tip, a rigid endoscope with improved and more even light distribution characteristics can be constructed.

**Claims**

1. An endoscope having a rigid insertion sheath (13) housing an eccentrically-positioned inner tube (17) containing an observing optical system, a bunch of light-guide fibres (21) for transmitting illuminating radiation to be projected from their end surfaces partially surrounding the outer periphery of the inner tube, characterised in that the inner tube (17) has a partially-curved tip surface made by cutting a first member (20) with an outer part-spherical end face to form an inclined viewing aperture and the outer sheath (13) also has a partially-curved tip surface made by cutting a second member (32) with an inner part-spherical surface to leave an inclined end face aperture that is substantially filled by said inner tube and said optical fibres.

2. An endoscope according to Claim 1, characterised in that said partially-curved tip surface of the outer sheath (13) is formed by cutting a hemispherical curved surface.

3. An endoscope according to Claim 1. modified in that said outer sheath (13) is formed by cutting a part-conical curved surface.

4. An endoscope according to Claim 1, modified in that said outer sheath (13) is formed by cutting a part-parabolic curved surface.

5. An endoscope according to Claim 2, characterised in that said inner tube (17) is formed by cutting a hemispherical curved surface.

6. An endoscope according to Claim 3, modified in that said inner tube (17) is formed by cutting a part-conical curved surface.

7. An endoscope according to Claim 4, modified in that said inner tube (17) is formed by cutting a part-parabolic curved surface.

8. An endoscope according to any preceding Claim, characterised in that said inner tube (17) has a saddle-shaped filler member (22) secured on its outer periphery, the inner and outer contours of which match the outer diameter of the inner tube and the inner diameter of the outer sheath (13).

**Patentansprüche**

1. Endoskop mit einer starren Einführungshülse (13), die ein exzentrisch angeordnetes inneres Rohr (17) aufnimmt, das ein optisches Beobachtungssystem enthält, sowie ein Bündel von Lichtleitfasern (21) zum Übertragen von Lichtstrahlen, die an deren Stirnflächen auftreten, die teilweise die äußere Peripherie des inneren Rohres umgeben, dadurch gekennzeichnet, daß das innere Rohr (17) eine teilweise gekrümmte Spitzenoberfläche aufweist, die durch Schneiden eines ersten Teiles (20) mit einer äußeren teilsphärischen Stirnfläche zur Bildung einer geneigten Betrachtungsöffnung entstanden ist und wobei die äußere Hülse (13) ebenfalls eine teilweise gekrümmte Oberfläche aufweist, die durch Schneiden eines zweiten Teiles (32) mit einer inneren teil-sphärischen Fläche entstanden ist, zur Bildung einer geneigten Stirnflächenöffnung, die im wesentlichen von dem inneren Rohr und den optischen Fasern ausgefüllt ist.

2. Endoskop nach Anspruch 1, dadurch gekennzeichnet, daß die teilweise gekrümmte Spitzenoberfläche der äußeren Hülse (13) durch Schneiden einer halbkugeligen Oberfläche gebildet ist.

3. Endoskop nach Anspruch 1, abgewandelt dadurch, daß die äußere Hülse (13) durch Schneider einer teil-konischen gekrümmten Oberfläche gebildet ist.

4. Endoskop nach Anspruch 1, abgewandelt dadurch, daß die äußere Hülse (13) durch Schneiden einer teil-parabolisch gekrümmten Oberfläche gebildet ist.

5. Endoskop nach Anspruch 2, dadurch gekennzeichnet, daß das innere Rohr (17) durch Schneiden einer halbkugelig gekrümmten Oberfläche gebildet ist.

6. Endoskop nach Anspruch 3, abgewandelt dadurch, daß das innere Rohr (17) durch Schneiden einer teil-konisch geformten Oberfläche gebildet ist.

7. Endoskop nach Anspruch 4, abgewandelt dadurch, daß das innere Rohr (17) durch Schneiden einer teil-parabolisch gekrümmten Oberfläche gebildet ist.

8. Endoskop nach einem der vorstehenden Ansprüche, dadurch gekennzeichnet, daß das innere Rohr (17) ein sattelähnlich geformtes Füllstück (22) befestigt an der äußeren Peripherie aufweist, wobei die innere und die äußere Kontur dem äußeren Durchmesser des inneren Rohres und dem inneren Durchmesser der äußeren Hülse (13) angepaßt sind.

**Revendications**

1. Endoscope comprenant une gaine d'insertion rigide (13) dans laquelle est placé un tube intérieur excentrique (17) contenant un système optique d'observation, un faisceau de fibres servant de guide lumineux (21) pour transmettre un rayonnement d'éclairage à projeter à partir de leur surface d'extrémité entourant partiellement la périphérie extérieure du tube intérieur, caractérisé par le fait que le tube intérieur (17) possède une surface d'extrémité partiellement courbée obtenue en découpant un premier élément (20) avec une face d'extrémité extérieure partiellement sphérique pour former une ouverture de visée inclinée et que la gaine extérieure (13) possède aussi une surface d'extrémité partiellement courbée obtenue en découpant un second élément (32) avec une partie intérieure de surface sphérique pour laisser une ouverture de face d'extrémité inclinée qui est sensiblement remplie par ledit tube intérieur et par lesdites fibres optiques.

2. Endoscope selon la revendication 1, caractérisé par le fait que ladite surface d'extrémité partiellement courbée de la gaine extérieure (13) est formée en découpant une surface courbe hémisphérique.

3. Endoscope selon la revendication 1, modifié par le fait que ladite gaine extérieure (13) est formée en découpant une surface courbe partiellement conique.

4. Endoscope selon la revendication 1, modifié par le fait que ladite gaine extérieure (13) est formée en découpant une surface courbe partiellement parabolique.

5. Endoscope selon la revendication 2, caractérisé par le fait que ledit tube intérieur est formé en découpant une surface courbe hémisphérique.

6. Endoscope selon la revendication 3, modifié par le fait que ledit tube intérieur (17) est formé en découpant une surface courbe partiellement conique.

7. Endoscope selon la revendication 4, modifié par le fait que ledit tube intérieur (17) est formé en découpant une surface courbe partiellement parabolique.

8. Endoscope selon l'une quelconque des revendications précédentes, caractérisé par le fait que ledit tube intérieur (17) comprend un élément de remplissage en forme de selle (22) fixé sur sa périphérie extérieure, dont les contours intérieurs et extérieurs correspondent au diamètre extérieur du tube intérieur et au diamètre intérieur de la gaine extérieure (13).

0  070  652

# FIG.1

# FIG.2

# FIG.3

# FIG.4

1

# FIG.5

( a )

( b )

( c )

# FIG.6

# FIG.7

# FIG.8

# FIG.9

( a )

17

20

( b )

20

17

( c )

17

20

# FIG.10

( a )

22

17

( b )

20

17

( c )

22

20

17

# FIG.11

16    22    17

21

23    20

# FIG.12

# FIG.13

# FIG.14

(a)

(b)

(c)

## FIG.15

## FIG.16

## FIG.17

## FIG.18